# EUROPEAN PATENT APPLICATION

(11) **EP 4 260 832 A1**
(43) Date of publication of application: **18.10.2023**
(21) Application number: 21902234.0
(22) Date of filing: 21.10.2021
(51) Int. Cl.: A61F 2/24

(54) **VALVE CLIP DELIVERY DEVICE**

(30) Priority: 11.12.2020 CN 202011445523; 11.12.2020 CN 202022998734 U
(71) Applicant: Hangzhou Valgen Medtech Co., Ltd., Hangzhou, Zhejiang 310051 (CN)
(72) Inventor: ZHANG, Tingchao, Hangzhou, Zhejiang 310051 (CN); LI, Liguang, Hangzhou, Zhejiang 310051 (CN); ZHANG, Weiwei, Hangzhou, Zhejiang 310051 (CN)
(74) Representative: Tansini, Elio Fabrizio
(86) International application number: PCT/CN2021/125369
(87) International publication number: WO 2022/121527

(57) **Abstract**

Some embodiments of the disclosure provide a valve clip delivery device. The valve clip includes at least two clamping members and is further provided with a self-locking mechanism. The valve clip delivery device includes a handle and a catheter, wherein the handle includes a housing, a clamping control assembly and a locking control assembly, wherein a proximal end of the catheter is fixedly connected with a distal end of the housing, the clamping control assembly and the locking control assembly are both disposed on the housing, the locking control assembly is connected with the self-locking mechanism and is configured to control the valve clip to be switched between an unlocked state and a self-locking state, and the clamping control assembly is configured to control the at least two clamping members to be opened or closed when the valve clip is in the unlocked state.

## Description

### Cross-Reference to Related Application

The present disclosure is a national stage application of International Patent Application No. PCT/CN2021/125369, which is filed on October 21, 2021, and claims priority to Chinese Patent Application No. 2020114455239 (entitled "valve clip delivery device") and 2020229987347 (entitled "valve clip delivery device"), and filed on December 11, 2020, the contents of which are hereby incorporated by reference in its entirety.

### Technical Field

The disclosure relates to a technical field of medical instruments, and in particular to a valve clip delivery device.

### Background

A mitral valve is a one-way valve located between a left atrium and a left ventricle of a heart. The mitral valve which is normal and healthy can control flow of blood from the left atrium to the left ventricle, and further avoid flow of blood from the left ventricle to the left atrium. The mitral valve includes a pair of leaflets, which are referred to as an anterior leaflet and a posterior leaflet. Under normal circumstances, during the systole of the left ventricle, edges of the anterior leaflet and the posterior leaflet are completely aligned, so as to prevent blood from flowing from the left ventricle to the left atrium. When the leaflets, chordae tendineae or annulus of the mitral valve is diseased, for example, such as partial rupture of the chordae tendineae and poor alignment of the anterior leaflet and the posterior leaflet of the mitral valve, the mitral valve cannot be fully closed during the systole of the left ventricle, such that blood flows back from the left ventricle to the left atrium, resulting in a series of pathological and physiological changes, which are referred to as "mitral regurgitation".

Edge-to-edge repair of the mitral valve is an effective method for treating mitral regurgitation. Specifically, the edges of the anterior leaflet and the posterior leaflet of the mitral valve that cannot be normally aligned are fixed together by means of suturing or clamping, so as to reduce a leaflet gap, such that a double orifice structure is formed at a valve orifice of the mitral valve, so as to reduce a total area of the valve orifice of the mitral valve and reduce or eliminate regurgitation. A traditional edge-to-edge repair surgery for a mitral valve is operated under direct vision conditions, and usually requires thoracotomy and establishment of extracorporeal blood circulation, resulting in a high risk. With advancement of a technology, various minimally invasive surgeries and interventional surgeries have been developed. These surgeries only require a small operating window on a body of a patient, and thus a valve clip is delivered to the diseased mitral valve by utilizing a delivery device. The valve clip clamps and fixes part of edges of two leaflets together, and the double orifice structure is formed at the valve orifice of the mitral valve, thereby reducing regurgitation. However, an existing valve clip delivery device typically allows a valve clip to be actuated at any position and at any moment, posing a risk that the valve clip is accidentally opened, thereby disrupting a surgical process.

### Summary

The technical problem to be solved by the disclosure is to provide a valve clip delivery device, which can avoid a risk of accidental opening of a valve clip.

In order to achieve the above objective, some embodiments of the disclosure use the following technical solution:
Some embodiments of the disclosure provide a valve clip delivery device configured to deliver and control a valve clip. The valve clip includes at least two clamping members which are relatively opened and closed, and the valve clip is provided with a self-locking mechanism. The valve clip delivery device includes a handle and a catheter, wherein the handle includes a housing, a clamping control assembly and a locking control assembly, wherein a proximal end of the catheter is fixedly connected with a distal end of the housing, the clamping control assembly and the locking control assembly are both disposed on the housing, the locking control assembly is connected with the self-locking mechanism and is configured to control the valve clip to be switched between an unlocked state and a self-locking state, and the clamping control assembly is configured to control the at least two clamping members to be opened or closed when the valve clip is in the unlocked state.

According to the valve clip delivery device provided by the disclosure, since the locking control assembly is arranged, it can be ensured that the valve clip is delivered in place in the self-locking state. Only after the valve clip is controlled to be switched to the unlocked state by means of the locking control assembly, the at least two clamping members of the valve clip can be opened to clamp leaflets such that the valve clip can be prevented from being accidentally opened in a delivery process, thereby being conducive to smooth operation of a valve edge-to-edge repair surgery.

### Brief Description of the Drawings

Fig. 1 illustrates a three-dimensional assembly schematic diagram of a valve clip system of some embodiments of the present disclosure;
Fig. 2 illustrates a three-dimensional schematic diagram of a base, a driving arm and a clamping member of a valve clip that are assembled together;
Fig. 3 illustrates a cross-sectional schematic diagram of a base, a driving arm and a clamping member of a valve clip that are assembled together;
Fig. 4 illustrates a schematic diagram of a self-locking mechanism of a valve clip;
Fig. 5 illustrates a three-dimensional assembly schematic diagram of a valve clip and a distal end of a valve clip delivery device;
Fig. 6 illustrates a three-dimensional schematic diagram of two grasping members of a valve clip opening radially and two clamping members of a valve clip opening radially;
Figs. 7, 8 and 9 are schematic diagrams of assembly of partial structures of a valve clip system;
Fig. 10 illustrates a three-dimensional assembly schematic diagram of a handle of a valve clip delivery device;
Fig. 11 illustrates a three-dimensional assembly schematic diagram of the handle having a housing partially removed in Fig. 10;
Fig. 12 illustrates a three-dimensional exploded schematic diagram of the handle in Fig. 10;
Fig. 13 illustrates a cross-sectional schematic diagram of a catheter;
Fig. 14 illustrates a three-dimensional assembly schematic diagram of a first control rod, a first sleeve and a first fixing member of a locking control assembly;
Fig. 15 illustrates an exploded schematic diagram of a first control rod, a first sleeve and a first fixing member of a locking control assembly;
Fig. 16 illustrates a schematic diagram of a three-dimensional structure of a first sleeve in the locking control assembly shown in Fig. 14;
Fig. 17 illustrates a schematic diagram of operation of a locking control assembly of a valve clip delivery device to switch a valve clip from an unlocked state to a self-locking state;
Fig. 18 illustrates a schematic diagram of operation of a locking control assembly of a valve clip delivery device to switch a valve clip from a self-locking state to an unlocked state;
Fig. 19 illustrates a three-dimensional assembly schematic diagram of a clamping control assembly and a disengagement control assembly;
Fig. 20 illustrates a three-dimensional exploded schematic diagram of a clamping control assembly;
Fig. 21 illustrates a schematic structural diagram of a constant-force gear of a clamping control assembly;
Fig. 22 illustrates a schematic structural diagram of a knob of a clamping control assembly;
Fig. 23 illustrates a schematic diagram of operation of a clamping control assembly of a valve clip delivery device to relatively open two clamping members of a valve clip;
Fig. 24 illustrates a schematic diagram of operation of a clamping control assembly of a valve clip delivery device to relatively close two clamping members of a valve clip;
Fig. 25 illustrates a schematic diagram of assembly of a second control rod, a second sleeve and a second fixing member of a grasping control assembly;
Fig. 26 illustrates an exploded schematic diagram of a second control rod, a second sleeve and a second fixing member of a grasping control assembly;
Fig. 27 illustrates a three-dimensional structure schematic diagram of a second sleeve of the grasping control assembly shown in Fig. 25;
Fig. 28 illustrates a schematic diagram of operation of two grasping control assemblies simultaneously controlling two grasping members to be opened;
Fig. 29 illustrates a schematic diagram of operation of two grasping control assemblies simultaneously controlling two grasping members to be relatively close to each other;
Fig. 30 illustrates a schematic diagram of operation of two grasping control assemblies controlling one grasping member to be opened and the other grasping member to abut against a shaft body of a valve clip respectively;
Fig. 31 illustrates a three-dimensional exploded schematic diagram of a disengagement control assembly and a transmission member;
Fig. 32 illustrates a three-dimensional schematic diagram of a disengagement control assembly and a transmission member that are assembled together; and
Fig. 33 illustrates a schematic diagram of operation of switching a disengagement control assembly from a first state to a second state.

### Detailed Description of the Embodiments

In order to make the objectives, technical solutions, and advantages of the disclosure clearer, the disclosure will be further described in detail below referring to the accompanying drawings.

It should be understood that expressions such as "include", "comprise", "may include" and "may comprise" that can be used in the disclosure indicate existence of disclosed functions, operations, or constituent elements, and do not limit one or more additional functions, operations, or constituent elements. In the present disclosure, terms such as "including", "comprise" and/or "having" can be interpreted as indicating a specific characteristic, number, operation, constituent element, assembly or combination thereof, but cannot be interpreted as excluding the existence or addition possibility of one or more other characteristics, number, operation, constituent element, assembly or combination thereof.

In addition, in the disclosure, the expression "and/or" includes any and all combinations of words listed in association. For example, the expression "A and/or B" can include A, B, or both A and B.

In the disclosure, expressions including ordinal numbers such as "first" and "second" can modify various elements. However, these elements are not limited by the above expression. For example, the above expression does not limit the order and/or importance of the elements. The above expression is only configured to distinguish one element from other elements. Similarly, without departing from the scope of the present disclosure, a first element can be referred to as a second element. Similarly, the second information can also be referred to as the first element.

When an assembly is referred to as "connecting" or "being connected with" other assemblies, it should be understood that the assembly not only directly connects or is connected with other assemblies, but another assembly can further exist between the assembly and other assemblies. On the other hand, when the assembly is referred to as "directly connecting" or "being directly connected with" other assemblies, it should be understood that there are no assemblies between the assemblies.

In the technical field of interventional medical instruments, a direction close to an operator is generally defined as a proximal end, and a direction away from the operator is defined as a distal end. The direction of a central axis of rotation of objects such as cylinders and tubes is defined as an axial direction. A circumferential direction refers to a direction (perpendicular to an axis and further perpendicular to a sectional radius) around the axis of a type of objects such as cylinders or tubes.

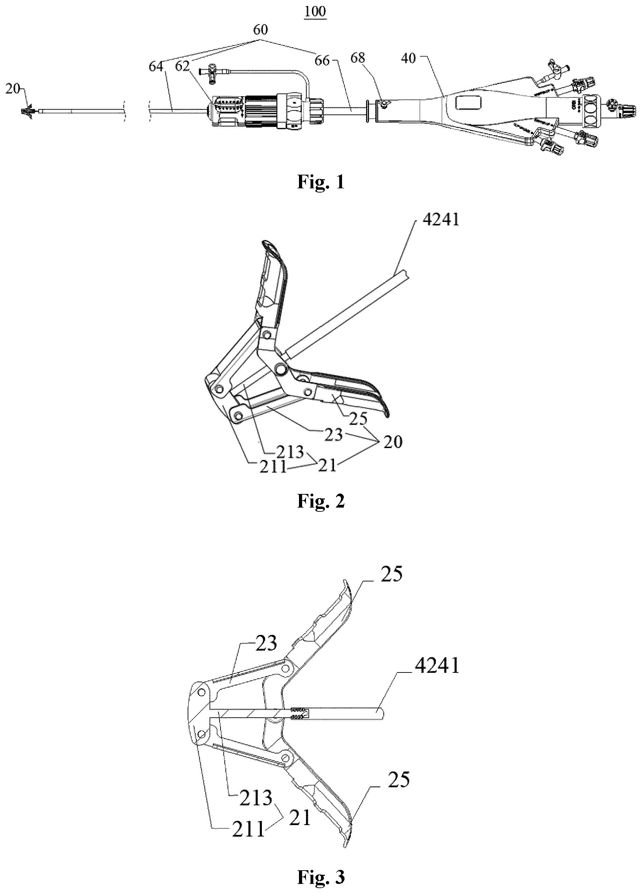

Referring to Fig. 1, a valve clip system 100 is provided in some embodiments of the disclosure. The valve clip system 100 is configured to perform edge-to-edge repair of a valve (not shown in the figure, which can be a mitral valve or a tricuspid valve). The valve clip system 100 includes a valve clip 20 and a valve clip delivery device 40. The valve clip 20 is detachably connected with a distal end of the valve clip delivery device 40, and is configured to clamp a plurality of leaflets of the valve, so as to implement edge-to-edge repair on the valve. The valve clip delivery device 40 is configured to deliver and control the valve clip 20.

Referring to Figs. 2 and 3, a valve clip 20 includes a base 21, two driving arms 23 rotatably connected to two sides of the base 21 respectively, and two clamping members 25. The two driving arms 23 and the two clamping members 25 are disposed in a one-to-one correspondingly manner. Each of the two clamping members 25 is rotatably connected with a corresponding driving arm 23 of the two driving arms 23, first ends of the two clamping members 25 are rotatably connected with each other by means of a pin (not shown), and second ends of the two clamping members 25 are both free ends. The base 21 is detachably connected with a distal end of the valve clip delivery device 40. The base 21, the two driving arms 23 and the two clamping members 25 form a linkage mechanism. The base 21 moves in an axial direction of the valve clip 20, such that the two driving arms 23 can drive the two clamping members 25 to be opened radially or closed, so as to clamp the valve.

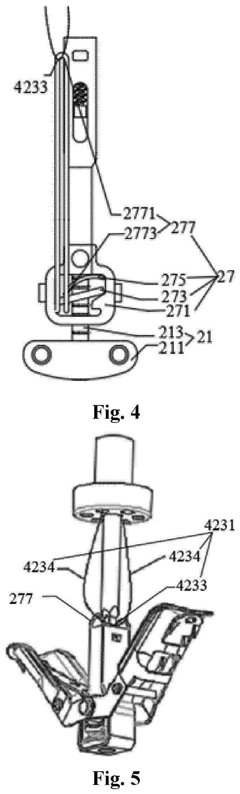

Referring to Fig. 4, a valve clip 20 further includes a self-locking mechanism 27 for self-locking the valve clip 20. A base 21 includes a base body 211 and a shaft body 213 protruding from the base body 211. Two sides of the base body 211 are each rotatably connected with one of the two clamping members 25. The self-locking mechanism 27 includes a frame body 271, a locking piece 273 and a resilient piece 275. The frame body 271 is sleeved outside the shaft body 213, and an axial position of the frame body 271 relative to the first ends of the two clamping members 25 is fixed. The locking piece 273 and the resilient piece 275 are accommodated in the frame body 271. The locking piece 273 is sleeved outside the shaft body 213, the resilient piece 275 is disposed between the frame body 271 and the locking piece 273, the resilient piece 275 is configured to bias the locking piece 273 to make the locking piece 273 inclined, and the locking piece 273 is provided with a through hole for the shaft body 213 to pass therethrough. When the locking piece 273 is inclined, an inner wall of the through hole is abutted against part of a side wall of the shaft body 213, such that the shaft body 213 can not move in its axial direction and rotate in its circumferential direction.

The valve clip 20 has an unlocked state and a self-locking state. The valve clip 20 can be switched between the unlocked state and the self-locking state. When the resilient piece 275 biases the locking piece 273, the through hole of the locking piece 273 is abutted against the shaft body 213 of the base 21, the shaft body 213 can not move in its axial direction relative to the locking piece 273, and the valve clip 20 is in the self-locking state. When the locking piece 273 is subjected to an external force to overcome a bias of the resilient piece 275, the locking piece 273 returns to a desired position, such that the inner wall of the through hole for the shaft body 213 to pass therethrough on the locking piece 273 is no longer abutted against the shaft body 213 of the base 21. The shaft body 213 can move in its axial direction relative to the locking piece 273, and the valve clip 20 is switched from the self-locking state to the unlocked state. In this case, the valve clip delivery device 40 can control the shaft body 213 to move in the axial direction, such that the two clamping members 25 can be driven to be opened radially or closed.

Referring to Figs. 4 and 5, the self-locking mechanism 27 further includes a connecting member 277. The connecting member 277 is connected between the locking piece 273 and the valve clip delivery device 40, and is configured to drive the locking piece 273 to overcome a bias of the resilient piece 275 under the driving of the valve clip delivery device 40, such that the valve clip 20 is switched from the self-locking state to the unlocked state. In some embodiments of the present disclosure, the connecting member 277 is U-shaped. The connecting member 277 includes a folded section 2771 and two connecting sections 2773 extending from two sides of the folded section 2771 respectively. The folded section 2771 is connected with the valve clip delivery device 40, and the connecting section 2773 is connected with the locking piece 273. Understandably, the connecting member 277 is not limited to a U-shape, and the connecting member 277 only needs to connect the locking piece 273 and the valve clip delivery device 40.

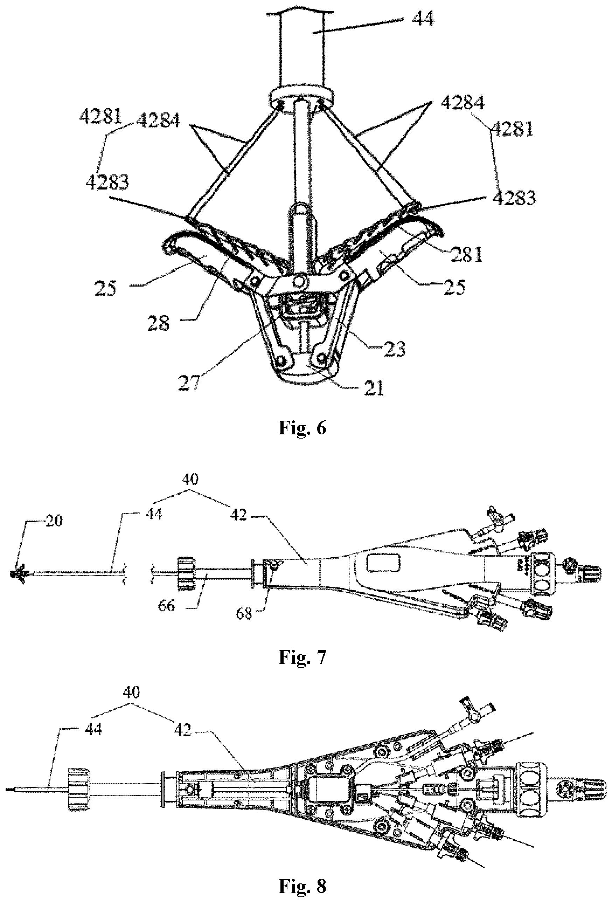

Referring to Fig. 6, the valve clip 20 further includes two grasping members 28 (or referred to as barbed elastic pieces). The two grasping members 28 and the two clamping members 25 are disposed in a one-to-one correspondingly manner, and the two grasping members 28 are configured to match the two clamping members 25 to clamp leaflets respectively. One end of each of the two grasping members 28 is close to a first end of a corresponding clamping member 25 of the two clamping members 25, and the other end of the each of the two grasping members 28 is connected with the valve clip delivery device 40. The each of two grasping members 28 has an elastic deformability, and the two grasping members 28 are opened radially without action of an external force. During an use of the valve clip system 100, after the leaflet is positioned between the grasping member 28 and the clamping member 25, the valve clip delivery device 40 controls the clamping member 25 to be closed, and the each of the two grasping members 28 matches the corresponding clamping member 25 to capture and position the leaflet. The grasping member 28 can further be provided with barbs 281 configured to press the leaflet to an inner surface of each of the two clamping members 25, so as to improve anchoring and maintenance of the leaflet.

Understandably, in some embodiments of the present disclosure, more than two clamping members 25 can be arranged, and more than two driving arms 23 can be arranged, just ensure that the valve clip 20 can clamp or release the leaflets, and the valve clip 20 can be in the self-locking state or the unlocked state.

Referring to Figs. 7 and 8, a valve clip delivery device 40 includes a handle 42 and a catheter 44. The handle 42 is configured to facilitate an operator in holding and controlling a valve clip 20. A proximal end of the catheter 44 is fixedly connected with a distal end of the handle 42, and a distal end of the catheter 44 extends to the valve clip 20.

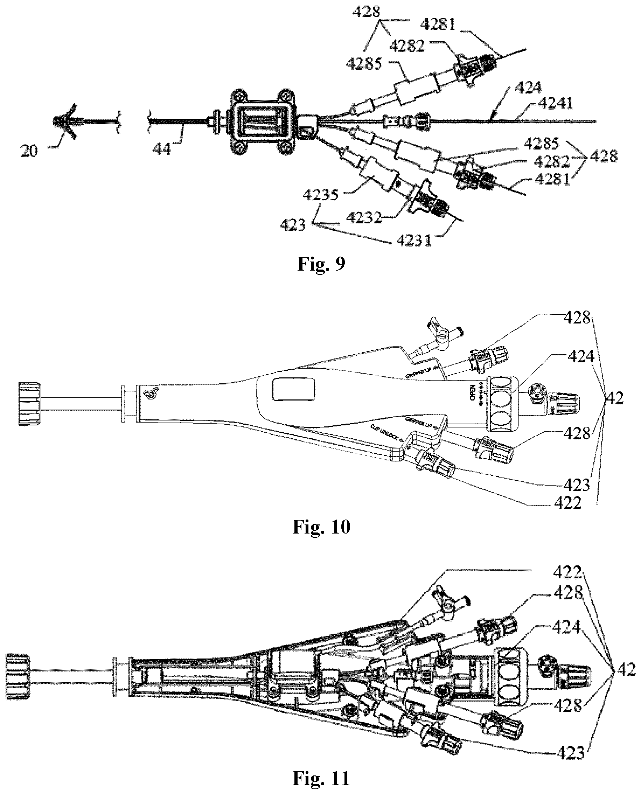

Referring to Figs. 9, 10 and 11, a handle 42 includes a housing 422, a locking control assembly 423, a clamping control assembly 424 and two grasping control assemblies 428. The locking control assembly 423, the clamping control assembly 424 and the two grasping control assemblies 428 are all disposed on the housing 422. The locking control assembly 423 is configured to control the valve clip 20 to be switched between the unlocked state and the self-locking state. The clamping control assembly 424 is configured to control the two clamping members 25 to be relatively opened or closed when the valve clip 20 is in the unlocked state. The two grasping control assemblies 428 and the two grasping members 28 are disposed in a one-to-one correspondingly manner. Each of the two grasping control assemblies 428 controls a corresponding grasping member 28 of the two grasping members 28.

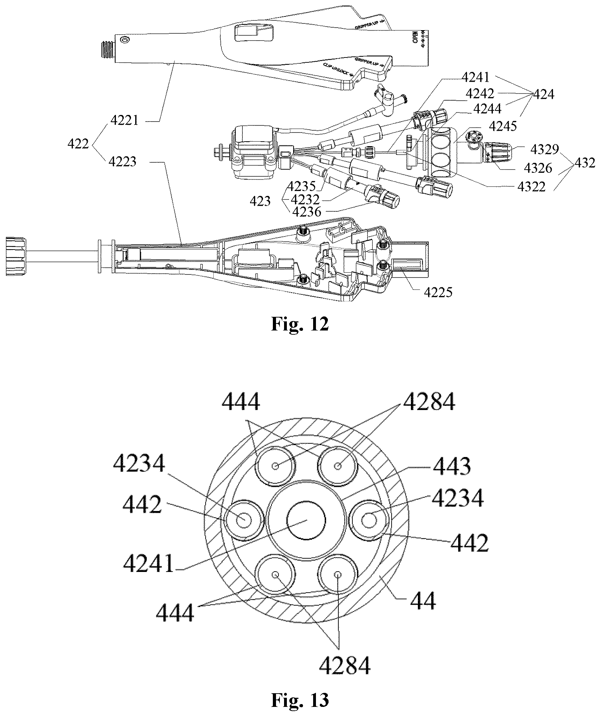

Referring to Fig. 12, in some embodiments of the present disclosure, a housing 422 includes a first housing 4221 and a second housing 4223 covering the first housing 4221. An accommodation space (not shown in the figure) is provided in the housing 422, and is configured to accommodate the clamping control assembly 424, the locking control assembly 423 and the two grasping control assemblies 428. A connection mode between the first housing 4221 and the second housing 4223 can be, but not limited to, buckling, threaded connection, etc.

Referring to Figs. 9 and 12, the locking control assembly 423 includes a locking control wire 4231 and a first control rod 4232. The first control rod 4232 is movably disposed in the housing 422. The locking control wire 4231 is configured to connect the first control rod 4232 and the locking piece 273 (as shown in Figs. 4 and 5) of the self-locking mechanism 27. A portion of the locking control wire 4231 located between the first control rod 4232 and the locking piece 273 of the self-locking mechanism 27 is movably disposed in the catheter 44. The first control rod 4232 drives the locking control wire 4231 to move in a direction from the distal end of the housing 422 to a proximal end of the housing 422, such that the valve clip 20 is switched from the self-locking state to the unlocked state.

The locking control assembly 423 in the valve clip system 100 according to present disclosure can ensure that the valve clip 20 is delivered in place in the self-locking state. Only after the valve clip 20 is controlled to be switched to the unlocked state by means of the locking control assembly 423, the clamping member 25 of the valve clip 29 can be opened to clamp the leaflets such that the valve clip 20 can be prevented from being accidentally opened in a delivery process, thereby being conducive to smooth operation of a valve edge-to-edge repair surgery, and ensuring safety of the surgery. In addition, the valve clip 20 can only be released after the valve clip being opened and then closed to clamp the leaflets. Therefore, the risk of accidental disengagement of the valve clip 20 is actually reduced by means of arrangement of the locking control assembly 423.

In some embodiments, the locking control wire 4231 passes through the first control rod 4232 along an axial direction of the first control rod 4232 and exposes a proximal end of the first control rod 4232. Referring to Fig. 5 anew, the locking control wire 4231 is U-shaped, and the locking control wire 4231 includes a folded section 4233 and two connecting sections 4234 extending from two sides of the folded section 4233 respectively.

Referring to Fig. 13, two first sub-cavities 442 are provided in the catheter 44, the two first sub-cavities 442 and the two connecting sections 4234 are disposed in a one-to-one correspondingly manner, and a proximal end of each of the two connecting sections 4234 movably passes through corresponding one of the two first sub-cavities 442 and is connected with the proximal end of the first control rod 4232. Referring to Fig. 4, the folded section 4233 of the locking control wire 4231 passes through the folded section 2771 of the connecting member 277, such that the locking control wire 4231 is movably connected with the self-locking mechanism 27.

Each of the two connecting sections 4234 passes through the corresponding one of the two first sub-cavities 442 to connect with the first control rod 4232 to make the locking control wire 4231 be U-shaped, such that the two connecting sections 4234 of the locking control wire 4231 can be prevented from being intertwined with each other, and the locking control wire 4231 can be withdrawn more smoothly and easily, thereby avoiding the risks of wire tearing and breakage, and improving the safety. Understandably, the connecting member 277 can be omitted, and the locking control wire 4231 is directly movably connected with the locking piece 273.

Since the locking control wire 4231 needs to react immediately when the first control rod 4232 is moved towards the proximal end of the housing 422, it is required for the locking control wire 4231 to have low ductility and high tensile breaking stress. In some embodiments of the disclosure, the locking control wire 4231 should have the tensile breaking stress greater than or equal to 25Mpa. The locking control wire 4231 can be made of at least one of ultra-high molecular weight polyethylene, nickel-titanium alloy, and stainless steel. In some embodiments of the disclosure, the locking control wire 4231 can be made of at least one of an ultra-high molecular weight polyethylene suture, a nickel-titanium wire, a stainless steel wire, a high molecular weight polymer wire, etc. In some embodiments of the disclosure, the locking control wire 4231 is made of the ultra-high molecular weight polyethylene suture.

Referring to Figs. 9, 12, 14 and 15, the locking control assembly 423 further includes a first sleeve 4235 and a first fixing member 4236. The first sleeve 4235 is fixedly disposed in the housing 422. A fixing method of the first sleeve 4235 fixed in the housing 422 can be snapping, or adhesion, or threaded connection, which is not limited herein. The first control rod 4232 is movably disposed in the first sleeve 4235. A first guide portion 4230 is disposed on an outer wall of the first control rod 4232.

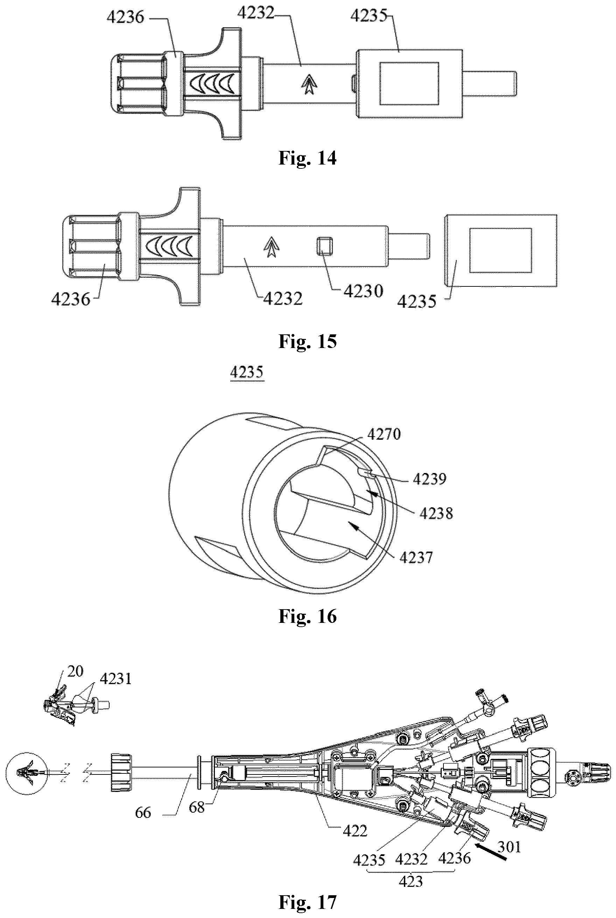

Referring to Fig. 16, a second guide portion 4237 is disposed on an inner wall of the first sleeve 4235. The first guide portion 4230 and the second guide portion 4237 are configured to guide a movement of the first control rod 4232 in an axial direction of the housing 422, so as to improve smoothness of the movement of the first control rod 4232 in the axial direction of the housing 422.

In some embodiments of the disclosure, the first guide portion 4230 is a guide protrusion, the second guide portion 4237 is a guide groove disposed on an inner wall of the first sleeve 4235, and a limiting groove 4238 communicating with the guide groove is further disposed at a proximal end of the first sleeve 4235. The limiting groove 4238 extends in a circumferential direction of the first sleeve 4235 and is provided with a limiting step 4239, and the limiting groove 4238 is substantially an arc-shaped groove. When the guide protrusion of the first control rod 4232 is abutted against a bottom surface of the limiting groove 4238, the first control rod 4232 cannot move towards the distal end of the housing 422. An initial end surface of the limiting groove 4238 is flush with a wall surface of the second guide portion 4237, and the limiting step 4239 is disposed between a terminational end surface 4270 of the limiting groove 4238 and the other wall surface of the second guide portion 4237. The limiting step 4239 is configured to limit the first guide portion 4230 in the limiting groove 4238, so as to prevent the first guide portion 4230 from detaching from the limiting groove 4238. When the first guide portion 4230 is disposed in the limiting groove 4238 and abutted against the limiting step 4239, the valve clip 20 is kept in the unlocked state.

Referring to Figs. 9, 12, 14 and 15, a first fixing member 4236 is detachably connected with a proximal end of the first control rod 4232, and is configured to fix a free end of the connecting section 4234 of the locking control wire 4231 to the proximal end of the first control rod 4232. In some embodiments of the present disclosure, the first fixing member 4236 is a nut, and the first fixing member 4236 is in threaded connection with the proximal end of the first control rod 4232. Part (the free end of the connecting section 4234) of the locking control wire 4231 exposed from the first control rod 4232 is fixedly clamped between an inner wall of the first fixing member 4236 and an outer wall of the proximal end of the first control rod 4232.

The free ends of the two connecting sections 4234 of the locking control wire 4231 away from the folded section 4233 can be wound around the proximal end of the first control rod 4232, and the first fixing member 4236 is screwed on the proximal end of the first control rod 4232, so as to press and fix the free ends of the two connecting sections 4234 of the locking control wire 4231 away from the folded section 4233. Under the condition that the first fixing member 4236 is screwed tightly, the locking control wire 4231 can pull the locking piece 273 back to a horizontal position by pulling the first control rod 4232 back towards the proximal end of the housing 422, thereby unlocking the shaft body 213. Correspondingly, the locking control wire 4231 is loosened by pushing the first control rod 4232 towards the distal end of the housing 422, and the locking piece 273 is inclined under the action of the resilient piece 275, such that the valve clip 20 is in the self-locking state. Under the condition that the first fixing member 4236 is removed, the locking control wire 4231 can be removed from a patient body by pulling the free end of one of the two connecting sections 4234 of the locking control wire 4231, so as not to obstruct subsequent disengagement of the valve clip 20.

The valve clip 20 can be switched to the self-locking state or the unlocked state by operating the first control rod 4232 to drive the locking control wire 4231. Referring to Figs. 16 and 17, the operation required to switch the valve clip 20 from the unlocked state to the self-locking state is: the first guide portion 4230 moves in the limiting groove 4238 towards the terminational end surface 4270 away from the limiting groove 4238 by rotating the first control rod 4232, the first guide portion 4230 is disengaged from the limiting groove 4238 and enters the second guide portion 4237, then the first control rod 4232 is driven to move towards a distal end (for example, in a direction indicated by an arrow 301 in Fig. 17), the locking control wire 4231 is in a relaxed state, the locking piece 273 is biased by the resilient piece 275, and the valve clip 20 is switched to the self-locking state.

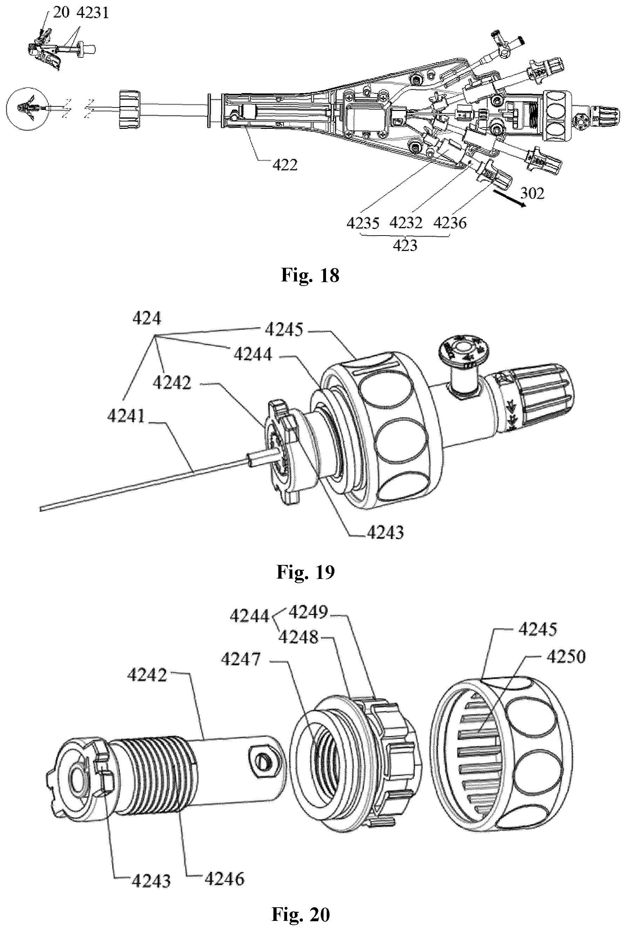

When the valve clip 20 needs to be switched from the self-locking state to the unlocked state, referring to Fig. 18, the first control rod 4232 is driven to move towards a proximal end (for example, in a direction indicated by an arrow 302 in Fig. 18), and the locking control wire 4231 is in a straightened state and pulls the connecting member 277, such that the locking piece 273 overcomes a bias of the resilient piece 275 and returns to a positive position, and the valve clip 20 is unlocked. After that, the first control rod 4232 is rotated to make the first guide portion 4230 enter the limiting groove 4238 and move towards the terminational end surface 4270 of the limiting groove 4238 until the first guide portion 4230 abuts against the limiting step 4239, and the first control rod 4232 pulls the locking control wire 4231 all the time to keep the valve clip 20 in the unlocked state, so as to allow the valve clip 20 to be opened or closed.

It can be understand that one of the first guide portion 4230 and the second guide portion 4237 is a guide groove extending in an axial direction of the first control rod 4232, the other one of the first guide portion 4230 and the second guide portion 4237 is a guide protrusion, and the guide protrusion is slidably disposed in the guide groove. Understandably, the first sleeve 4235 can be omitted, and the second guide portion can be directly disposed on the housing 422.

Understandably, the first fixing member 4236 is not limited to a nut, and can also be a cover. The first fixing member 4236 can be omitted, and a connection method by which the portion of the locking control wire 4231 exposed from the proximal end of the first control rod 4232 connects the first control rod 4232 is not limited. For example, the portion of the locking control wire 4231 exposed from the proximal end of the first control rod 4232 is directly wound around the first control rod 4232. Alternatively, the portion of the locking control wire 4231 exposed from the proximal end of the first control rod 4232 is directly fixed to the first control rod 4232 by means of bonding, etc. After the first control rod 4232 is pulled back towards the proximal end, the locking control wire 4231 is cut off, and then one strand of the locking control wire 4232 is pulled out. So the locking control wire 4232 can also be removed from the patient body, so as not to obstruct disengagement of the valve clip 20.

It should be noted that control of a distance of linear motion of the first control rod 4232 relative to the housing 422 should not only consider an optimal unlocking force value that the locking control wire 4231 can generate for the valve clip 20, but also consider loss of the locking control wire 4231 in the first sub-cavity 442 of the catheter 44 and an effect of extensibility of the locking control wire 4231. With the optimal unlocking force value is 6N as an example, the distance of linear motion of the first control rod 4232 relative to the housing 422 is set to [9, 13] mm. Thus, the valve clip 20 can be unlocked by pulling the first control rod 4232 backwards towards the proximal end, such that the situation that the valve clip 20 and the locking control wire 4231 are damaged due to an excessive pulling force is avoided. The valve clip 20 can be self-locked by pushing the first control rod 4231 forwards towards the distal end, such that it is ensured that a stacking amount of the locking control wire 4231 is not too large to cause the locking control wire 4231 to hang the valve clip 20. Understandably, the stroke of linear motion of the first control rod 4162 relative to the housing 422 is not limited in the disclosure.

Understandably, the structure and arrangement of the self-locking mechanism 27 of the valve clip 20 and the structure and arrangement of the locking control assembly 423 are not limited in the disclosure.

Referring to Figs. 9 and 12, the clamping control assembly 424 comprises a mandrel 4241 and a transmission member 4242. A distal end of the mandrel 4241 is detachably connected with a proximal end of the shaft body 213 (shown in Figs. 2 and 3) of the valve clip 20. In some embodiments, the distal end of the mandrel 4241 is in threaded connection with the proximal end of the shaft body 213. Understandably, it is not limited that the distal end of the mandrel 4241 is in threaded connection with the proximal end of the shaft body 213, and the distal end of the mandrel 4241 is connected with the proximal end of the shaft body by other methods, such as snapping, which is not limited herein. A proximal end of the mandrel 4241 movably passes through the catheter 44 and is connected with the transmission member 4242. The transmission member 4242 is slidably connected with the housing 422 in the axial direction of the housing 422. Referring to Fig. 13 anew, a central cavity 443 is further provided in a catheter 44, and the mandrel 4241 is movably disposed in the central cavity 443.

In some embodiments of present disclosure, referring to Figs. 19 and 20, a first guide structure 4243 is further disposed at a distal end of the transmission member 4242, and a second guide structure 4225 (as shown in Fig. 12) is disposed on the first housing 4221 of the housing 422 corresponding to the first guide structure 4243. The first guide structure 4243 is slidably connected with the second guide structure 4225 in the axial direction of the housing 4221, so as to guide axial motion of the transmission member 4242 relative to the housing 422, such that the transmission member 4242 can only move in the axial direction relative to the housing 422 but may not rotate. In some embodiments, the first guide structure 4243 is a guide groove, and the second guide structure 4225 is a guide rail extending in the axial direction of the housing 4221. Understandably, the first guide structure 4243 and the second guide structure 4225 are not limited in the disclosure. For example, in other embodiments, the first guide structure 4243 can be a guide rail, and the second guide structure 4225 can be a guide groove.

The clamping control assembly 424 further includes a constant-force gear 4244 and a knob 4245 driving the constant-force gear 4244 to rotate. The constant-force gear 4245 is in threaded connection with the transmission member 4242. The constant-force gear 4244 rotates to drive the transmission member 4242 to move in the axial direction of the housing 4221. That is, rotation of the constant-force gear 4244 is converted into axial motion of the transmission member 4242 relative to the housing 422, so as to control opening and closing of the two clamping members 25.

In some embodiments, an outer wall of the transmission member 4242 includes a screw section 4246, an inner wall of the constant-force gear 4244 is provided with a transmission thread 4247, and the screw section 4246 is matched to the transmission thread 4247 disposed in the constant-force gear 4244.

The knob 4245 is sleeved on the constant-force gear 4244. When the knob 4245 rotates towards a positive direction and a driving force of the knob 4245 to the constant-force gear 4244 is less than a preset force value, the knob 4245 drives the constant-force gear 4244 to synchronously rotate towards the positive direction, such that the transmission member 4242 moves towards one end of the housing 4221 in the axial direction relative to the housing 422. For example, the knob 4245 drives the constant-force gear 4245 to rotate towards the positive direction, and the transmission member 4242 and the mandrel 4241 move towards the proximal end in the axial direction, so as to drive the two clamping members 25 of the valve clip 20 to be relatively closed. The greater a distance of motion of the transmission member 4242 towards the proximal end is, the tighter a degree of closing of the two clamping members 25 is.

When the knob 4245 rotates towards the positive direction and the driving force of the knob 4245 to the constant-force gear 4244 is greater than or equal to the preset force value, the knob 4245 independently rotates relative to the constant-force gear 4244. For example, after the valve is clamped by the two clamping members 25, if the knob 4245 continues being rotated, the knob 4245 independently rotates in a sliding manner relative to the constant-force gear 4244, thereby preventing the clamping members 25 from excessively clamping the leaflets to damage the leaflets. Even if a driving force of the knob 4245 to the constant-force gear 4244 is excessively large, once the driving force exceeds the preset force value, an acting force applied to the mandrel 4241 can not be increased, such that the mandrel 4241 is prevented from being broken or damaged due to an excessive pulling force.

When the knob 4245 rotates towards an opposite direction of the positive direction, the knob 4245 drives the constant-force gear 4244 to synchronously rotate in the opposite direction of the positive direction, such that the transmission member 4242 slides towards an opposite end in the axial direction relative to the housing 422. For example, the knob 4245 drives the constant-force gear 4245 to rotate in the opposite direction of the positive direction, and the transmission member 4242 and the mandrel 4241 move towards the distal end in the axial direction, so as to drive the two clamping members 25 of the valve clip 20 to be relatively opened. The greater a distance of motion of the transmission member 4242 towards the distal end is, the greater a relative opening angle of the two clamping members 25 is.

The constant-force gear 4244 includes a gear body 4248 and a plurality of toothed arms 4249 protruding from an outer wall of the gear body 4248 and disposed at intervals in a circumferential direction of the gear body 4248, and an inner wall of the knob 4245 is provided with a plurality of stop portions 4250 matching the plurality of toothed arms 4249 in the circumferential direction of the gear body 4248; and the preset force value is a value of an acting force applied to the constant-force gear 4244 by the knob 4245 when each toothed arm 4249 of the plurality of toothed arms 4249 relatively slides through each corresponding stop portion 4250 of the plurality of stop portions 4250.

One end of the toothed arm 4249 is fixedly connected with the outer wall of the gear body 4248, and the other end of the toothed arm 4249 is a free end. At least part of the outer wall of the toothed arm 4249 extends in the circumferential direction of the gear body 4248.

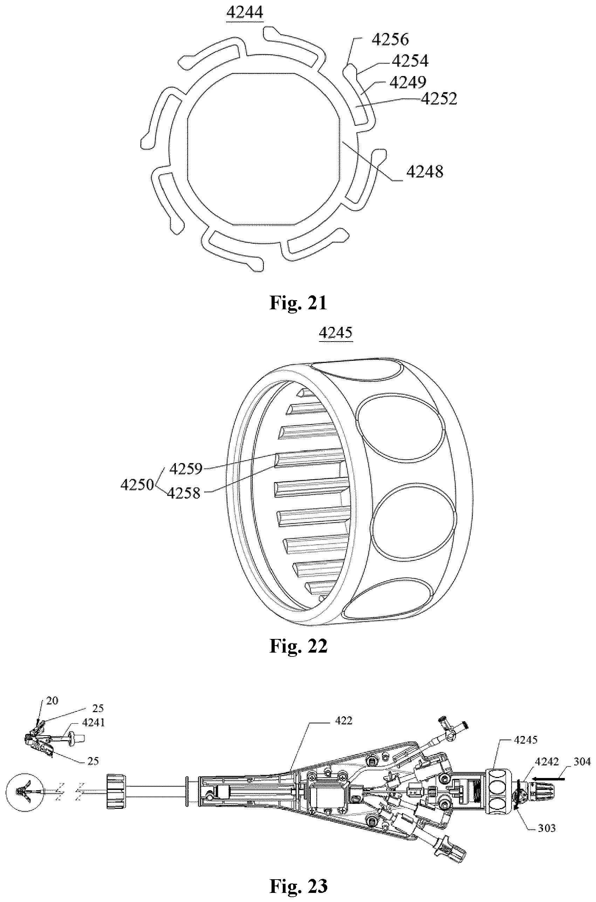

Referring to Fig. 21, a gap 4252 is formed between at least part of the outer wall of the toothed arm 4249 and the outer wall of the gear body 4248, thereby providing a deformation space for elastic deformation of the toothed arm 4249.

The preset force value can be controlled by configuring a maximum outer diameter of the constant-force gear 4244, a thickness of the toothed arm 4249, a material of the constant-force gear 4244, etc. In some embodiments, 8 toothed arms 4249 are disposed in the circumferential direction of the gear body 4248, the preset force value is about 50 N, the material of the constant-force gear 4244 is preferably polycarbonate (PC) having high hardness and toughness, the maximum outer diameter value is configured as 35 mm-40 mm, and the thickness value of the toothed arm 4249 is configured as 1.5 mm-2.0 mm. When a linear pulling force value applied to the mandrel 4241 is greater than a certain force value, the knob 4245 and the constant-force gear 4244 slide relatively. Even if the driving force of the knob 4245 to the constant-force gear 4244 is excessively large, once the driving force exceeds the preset force value, the mandrel 4241 stops moving in the axial direction, and an acting force applied to the mandrel 4241 is not increased.

A free end of the toothed arm 4249 includes a first sliding guide surface 4254 and a first sliding stop surface 4256 disposed opposite the first sliding guide surface 4254. Referring to Fig. 22, a stop portion 4250 of the knob 4245 includes a second sliding guide surface 4258 and a second sliding stop surface 4259 disposed opposite the second sliding guide surface 4258. When the knob 4245 rotates towards the positive direction, the first sliding guide surface 4254 is contacted with the second sliding guide surface 4258, such that when the knob 4245 rotates towards the positive direction and the driving force of the knob 4245 to the constant-force gear 4244 is greater than or equal to the preset force value, the toothed arm 4249 of the knob 4245 slides through the stop portion 4250 conveniently. When the knob 4245 rotates towards the opposite direction of the positive direction, the first sliding stop surface 4256 is contacted with the second sliding stop surface 4259, such that the constant-force gear 4244 rotates in the opposite direction of the positive direction along with the knob 4245.

In some embodiments, the first sliding guide surface 4254 is inclined relative to the first sliding stop surface 4256, the first sliding stop surface 4256 substantially extends in a radial direction of the gear body 4248. The second sliding guide surface 4258 is inclined relative to the first sliding stop surface 4259, and the second sliding stop surface 4259 substantially extends in a radial direction of the knob 4245. Contact between the first sliding guide surface 4254 and the second sliding guide surface 4258 is inclined surface contact, and the second sliding guide surface 4258 presses the first sliding guide surface 4254 towards the gap 4252, such that relative sliding between the first sliding guide surface 4254 and the second sliding guide surface 4258 is easy. Contact between the first sliding stop surface 4256 and the second sliding stop surface 4259 is substantially straight surface contact, and the second sliding stop surface 4259 pushes the first sliding stop surface 4256 towards a direction away from the gap 4252. Therefore, relative sliding between the first sliding stop surface 4256 and the second sliding stop surface 4259 is difficult. Understandably, in some embodiments, different degrees of roughness of surfaces are configured, such that relative sliding between the first sliding guide surface 4254 and the second sliding guide surface 4258 can be easy, and relative sliding between the first sliding stop surface 4256 and the second sliding stop surface 4259 can be difficult. For example, the degree of roughness of the first sliding guide surface 4254 can be less than that of the first sliding stop surface 4256, and the degree of roughness of the second sliding guide surface 4258 can be less than the degree of roughness of the second sliding stop surface 4259.

Referring to Fig. 23, when the valve clip 20 is in the unlocked state, the knob 4245 is rotated in the opposite direction (for example, a direction indicated by an arrow 303 in Fig. 23), and the transmission member 4242 drives the mandrel 4241 to move towards the distal end in the axial direction (for example, a direction indicated by an arrow 304 in Fig. 23), such that the two clamping members 25 of the valve clip 20 are relatively opened. That is, the two clamping members 25 of the valve clip 20 are opened radially.

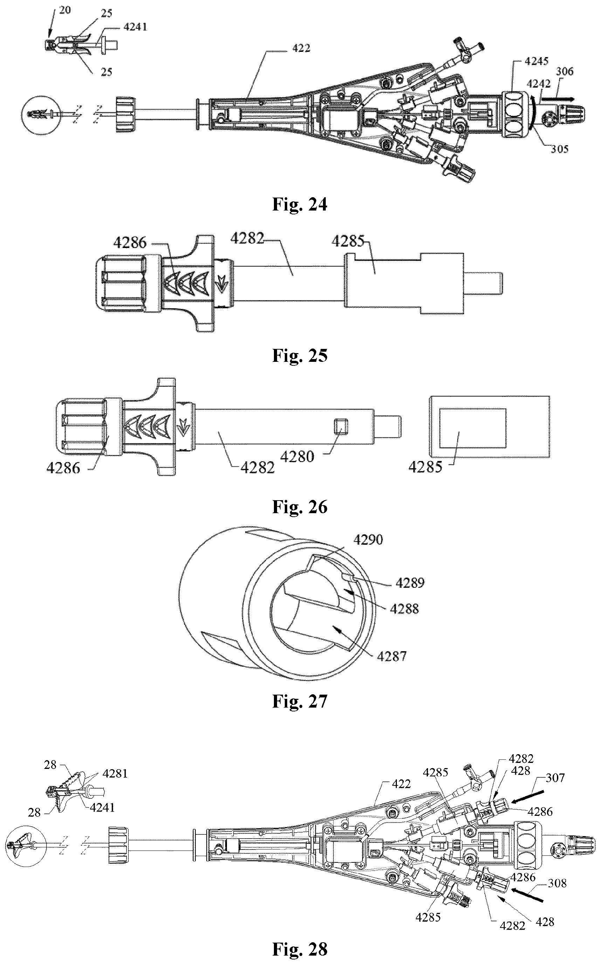

Referring to Fig. 24, when the valve clip 20 is in the unlocked state, the knob 4245 is rotated towards the positive direction (for example, a direction indicated by an arrow 305 in Fig. 24), and the transmission member 4242 drives the mandrel 4241 to slide towards the proximal end in the axial direction (for example, a direction indicated by an arrow 306 in Fig. 24), such that the two clamping members 25 of the valve clip 20 are relatively closed. That is, the two clamping members 25 of the valve clip 20 are relatively close to each other.

Referring to Figs. 9, 11 and 12, the two grasping control assemblies 428 are located at opposite sides of the housing 422 and opposite sides of the catheter 44 (for example, a central axis of the catheter 44), each grasping control assembly 428 is configured to control one grasping member 28 at the corresponding side and easy to distinguish. Each of the two grasping control assemblies 428 is similar to a locking control assembly 423 in structure. Each of the two grasping control assemblies 428 includes a grasping wire 4281 and a second control rod 4282. The second control rod 4282 is movably disposed in the housing 422. The grasping wire 4281 is correspondingly connected with the second control rod 4282 and the grasping member 28. A portion of the grasping wire 4281 located between the second control rod 4282 and the grasping member 28 is movably disposed in the catheter 44. Understandably, the number of grasping members 28 and the number of grasping control assemblies 428 are not limited in the disclosure. In some embodiments, more than two grasping members 28 can be disposed, more than two grasping control assemblies 428 can be disposed, and each grasping control assembly 428 correspondingly controls one of the grasping members 28.

Referring to Fig. 6 anew, the grasping wire 4281 is U-shaped and includes a folded section 4283 and two connecting sections 4284 extending from two sides of the folded section 4283 respectively. Referring to Fig. 13 anew, four second sub-cavities 444 are provided in the catheter 44, a proximal end of one of the two connecting sections 4284 correspondingly movably passes through one of the four second sub-cavities 444 and is connected with the second control rod 4282, and the folded section 4283 is movably connected with the grasping member 28.

Each of the two connecting sections 4284 passes through the corresponding one of the four second sub-cavities 444 to connect with the second control rod 4282 to make the locking control wire 4231 be U-shaped, such that the two second connecting sections 4284 of the grasping wire 4281 can be prevented from being intertwined with each other, and the grasping wire 4281 can be withdrawn more smoothly and easily, thereby avoiding the risks of wire tearing and breakage, and improving safety.

A stroke of the second control rod 4282 should be not less than 2πR×θ/360. R is a length of each of the two grasping members 28, θ is an absolute value of a difference value between a first angle and a second angle, the first angle is an angle between each of the two grasping members 28 and a central axis (a central axis of the shaft body 213) of the valve clip 20 when the two grasping members 28 are relatively close to each other, and the second angle is a maximum angle between one of the two grasping members 28 and the central axis of the valve clip 20 after the two grasping members 28 are opened radially. The first angle between each of the two grasping members 28 and the central axis of the valve clip 20 can be 0 degree when the two grasping members 28 are relatively close (or before released) to each other. After the two grasping members 28 are opened radially (or after released), the second angle between one of the two grasping members 28 and the central axis of the valve clip 20 can be less than 90 degrees, equal to 90 degrees, or greater than 90 degrees.

In consideration of loss of the grasping wire 4281 in the second sub-cavity 444, an effective advancing stroke of the second control rod 4282 is preferably configured as 2×(2πR×θ/360). For example, before the two grasping members 28 are relatively close to each other, a circumference of an arc caused by a movement of a free end of each of the two grasping members 28 is 13mm, and an effective stroke of the second control rod 4282 is preferably 25±2 mm. On one hand, when the two grasping members 28 are tightened, the grasping wire 4281 can not be broken or damaged. On the other hand, when the two grasping members 28 are released, the grasping wire 4281 can not be stacked too much.

Referring to Figs. 25 and 26, the grasping control assembly 428 further includes a second sleeve 4285 and a second fixing member 4286. The second sleeve 4285 is fixedly disposed in the housing 422. A fixing method of the second sleeve 4285 fixed in the housing 422 can be snapping, bonding, or threaded connection, which is not limited herein. The second control rod 4282 is movably disposed in the second sleeve 4285. A third guide portion 4280 is disposed on an outer wall of the second control rod 4282, and a fourth guide portion 4287 (as shown in Fig. 27) is disposed on an inner wall of the second sleeve 4285. The third guide portion 4280 and the fourth guide portion 4287 are configured to guide axial motion of the second control rod 4282 relative to the housing 422, so as to improve smoothness of axial motion of the second control rod 4282 relative to the housing 422.

In some embodiments, the third guide portion 4280 is a guide protrusion, and the fourth guide portion 4287 is a guide groove provided in an inner wall of the second sleeve 4285. A limiting groove 4288 in communication with the guide groove is further provided at a proximal end of the second sleeve 4285, the limiting groove 4288 extends in a circumferential direction of the second sleeve 4285 and is provided with a limiting step 4289, and the limiting groove 4288 is substantially an arc-shaped groove. When the guide protrusion of the second control rod 4282 is abutted against a bottom surface of the limiting groove 4288, the second control rod 4282 can not move towards the distal end relative to the second sleeve 4285. An initial end surface of the limiting groove 4288 is flush with a wall surface of third guide portion 4280, and the limiting step 4289 is disposed between a terminational end surface 4290 of the limiting groove 4288 and the other wall surface of the fourth guide portion 4287. The limiting step 4289 is configured to limit the third guide portion 4280 in the limiting groove 4288, so as to prevent the third guide portion 4280 from detaching from the limiting groove 4288. When the third guide portion 4280 disposed in the limiting groove 4288 and abuts against the limiting step 4289, the grasping wire 4281 is in a straightened state, and the grasping wire 4281 pulls the grasping member 28.

Understandably, one of the third guide portion 4280 and the fourth guide portion 4287 is a guide groove extending in an axial direction of the second control rod 4282, the other one of the third guide portion 4280 and the fourth guide portion 4287 is a guide protrusion, and the guide protrusion is slidably disposed in the guide groove. A position of the limiting groove 4288 is not limited. Understandably, the second sleeve 4285 can be omitted, and the second control rod 4282 can move in the axial direction of the housing 422.

The second fixing member 4286 is fixed to a proximal end of the second control rod 4282, and a portion of the grasping wire 4281 exposed from the second control rod 4282 is fixed to the proximal end of the second control rod 4282 by means of the second fixing member 4286. In some embodiments, the second fixing member 4286 is a nut, and the second fixing member 4286 is in threaded connection with the proximal end of the second control rod 4282. The portion of the grasping wire 4281 exposed from the second control rod 4282 is fixedly clamped between an inner wall of the second fixing member 4286 and an outer wall of the proximal end of the second control rod 4282.

The free ends of the two connecting sections 4284 of the grasping wire 4281 away from the folded section 4283 can be wound around the proximal end of the second control rod 4282, and the second fixing member 4286 is screwed on the second control rod 4282 to press and fix the free ends of the two connecting sections 4284 of the grasping wire 4281 away from the folded section 4283. Under the condition that the second fixing member 4286 is screwed tightly, the grasping wire 4281 can pull the grasping member 28 by pulling the second control rod 4282 back towards the proximal end, such that a leaflet accommodation space is formed between the grasping member 28 and the clamping member 25. Correspondingly, the grasping wire 4281 is loosened by pushing the second control rod 4282 towards the distal end, and under the elastic action of the grasping member 28, one end of the grasping member 28 connected with the grasping wire 4281 abuts against the clamping member 25, so as to capture the leaflet. Under the condition that the second fixing member 4286 is removed, the grasping wire 4281 can be removed from the patient body by pulling the free end of one of the two connecting sections 4284 of the grasping wire 4281, so as not to obstruct subsequent disengagement of the valve clip 20.

The grasping wire 4281 can be made at least one of a nickel-titanium wire, stainless steel wire, a high molecular polymer suture. In some embodiments, the grasping wire is made of a nickel-titanium wire having high strength, so as to reduce the risk of wire breakage.

Understandably, a second fixing member 4236 is not limited to a nut, and can also be a cover. The second fixing member 4286 can be omitted, and a connection mode between a portion of the grasping wire 4281 exposed from the proximal end of the second control rod 4282 and the second control rod 4282 is not limited. In some embodiments, the portion of the grasping wire 4281 exposed from the proximal end of the second control rod 4282 is directly wound around the second control rod 4282. In some embodiments, the portion of the grasping wire 4281 exposed from the proximal end of the second control rod 4282 is directly fixed to the second control rod 4282 by means of bonding, etc. After the second control rod 4282 is pulled back towards the proximal end, the grasping wire 4281 is cut off, and then one strand of the grasping wire 4281 is pulled out. Moreover, the grasping wire 4281 can also be removed from a patient body, so as not to obstruct disengagement of the valve clip 20.

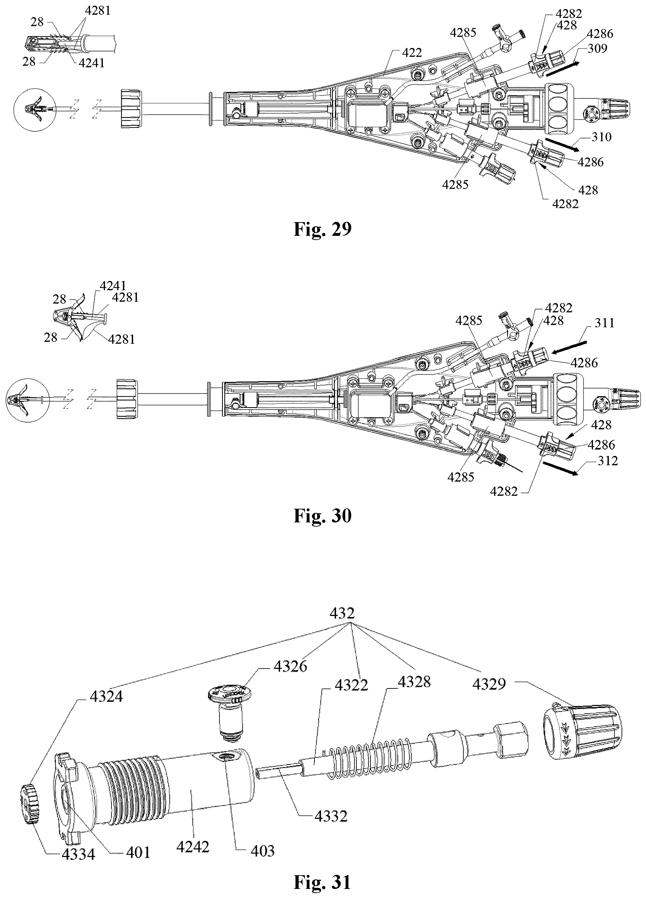

By operating the second control rod 4232, motion of the grasping member 28 can be controlled, so as to cooperate with the clamping member 25 to clamp or loose a leaflet. The two grasping control assemblies 428 can be simultaneously controlled. In some embodiments, referring to Fig. 28, the second control rods 4282 of the two grasping control assemblies 428 can be synchronously operated to move from the proximal end to the distal end (for example, directions indicated by an arrow 307 and an arrow 308 in Fig. 28), the two grasping wires 4281 are both in a relaxed state, and under the elastic action of each of the two grasping members 28, one end of each of the two grasping members 28 connected with the grasping wire 4281 abuts against the clamping member 25, so as to match the clamping member 25 to capture the leaflet conveniently. In some embodiments, referring to Fig. 29, the two second control rods 4282 of the two grasping control assemblies 428 can be synchronously operated to move from the distal end to the proximal end (for example, a direction indicated by an arrow 309 and an arrow 310 in Fig. 29), such that each grasping wire 4281 pulls corresponding grasping members 28 to be close towards the mandrel 4241, such that an accommodation space (not shown in the figure) for leaflet is formed between the grasping member 28 and the clamping member 25, and the leaflet can enter into the accommodation space. In some embodiments, one of the two grasping members 28 can also be independently controlled to capture a dynamic anterior leaflet or posterior leaflet on one side, and after the dynamic anterior leaflet or posterior leaflet is successfully captured, the other one of the two grasping members 28 can be controlled to capture the dynamic posterior leaflet or anterior leaflet on the other side, such that a difficulty of capturing the leaflet can be reduced, and capturing efficiency and a success rate of a surgery can be improved. For example, referring to Fig. 30, the second control rod 4282 of one of the two grasping control assemblies 428 moves from the proximal end to the distal end (for example, a direction indicated by an arrow 311 in Fig. 30), and the second control rod 4282 of the other one of the two grasping control assemblies 428 moves from the distal end to the proximal end (for example, a direction indicated by an arrow 312 in Fig. 30).

Referring to Figs. 11 and 12 anew, the valve clip delivery device 40 further includes a disengagement control assembly 432 configured to disengage the valve clip 20 from the mandrel 4241 after the valve clip 20 clamps and fixes the anterior leaflet and the posterior leaflet of the mitral valve to complete double-orifice treatment.

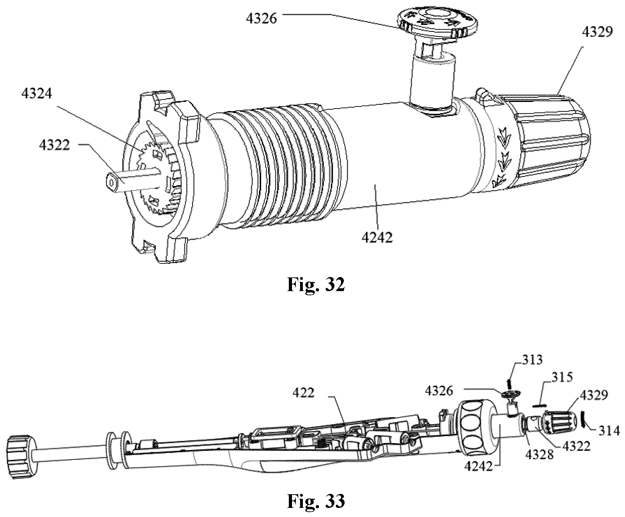

Referring to Figs. 31 and 32, the disengagement control assembly 432 includes a core rod 4322, a one-way gear 4324 and a safety switch 4326. The core rod 4322 is movably disposed in the transmission member 4242 and is fixedly connected with a proximal end of the mandrel 4241, and the one-way gear 4324 is fixedly sleeved on the core rod 4322 and accommodated in the transmission member 4242. The one-way gear 4324 only allows the core rod 4322 to drive the mandrel 4241 to unidirectionally rotate, so as to release connection between the mandrel 4241 and the shaft body 213 of the valve clip 20, such that the valve clip 20 is disengaged from the mandrel 4241.

In some embodiments, a distal end of the mandrel 4241 is in threaded connection with a proximal end of the shaft body 213. At least one first rotation stopping surface 4332 is disposed at a distal end of the core rod 4322, and a second rotation stopping surface 4334 is disposed on an inner wall of the one-way gear 4324 corresponding to the first rotation stopping surface 4332. The first rotation stopping surface 4332 matches the second rotation stopping surface 4334 to prevent relative rotation between the one-way gear 4324 and the core rod 4322.

The transmission member 4242 is provided with a hole 401 along an axial direction, and the core rod 4322 is disposed in the hole 401. A connecting hole 403 in communication with the hole 401 is provided on an outer wall of the transmission member 4342. The safety switch 4326 is disposed in the connecting hole 403.

The disengagement control assembly 432 has a first state and a second state. When the disengagement control assembly 432 is in the first state, the safety switch 4326 is abutted against the core rod 4322, and the core rod 4322 and the transmission member 4242 are connected into a whole; and when the disengagement control assembly 432 is in the second state, the safety switch 4326 is separated from the core rod 4322, and the core rod 4322 is driven to unidirectionally rotates relative to the transmission member 4242, thereby driving the distal end of the mandrel 4241 to be disengaged from the valve clip 20.

The disengagement control assembly 432 further includes an elastic member 4328. The elastic member 4328 extends along the axial direction and is held between the core rod 4322 and the transmission member 4242. In some embodiments, the elastic member 4328 is sleeved on the core rod 4322 and accommodated in the hole 401.

The disengagement control assembly 432 further includes a release knob 4329. The release knob 4329 is fixed to the proximal end of the core rod 4322 to rotate the core rod 4322 conveniently.

When the disengagement control assembly 432 needs to be switched from the first state to the second state, the safety switch 4326 is pulled up towards a direction away from the mandrel 4241 (for example, a direction indicated by an arrow 313 in Fig. 33), and the safety switch 4326 is separated from the core rod 4322. When the disengagement control assembly 432 is in the second state, the release knob 4329 can be unidirectionally rotated (for example, a direction indicated by an arrow 314 in Fig. 33), the transmission member 4242 is stationary relative to the housing 422, and the release knob 4329 drives the core rod 4322 and the mandrel 4241 to rotate relative to the transmission member 4242. Once the distal end of the mandrel 4241 is disengaged from the shaft body 213 of the valve clip 20, the pre-compressed elastic member 4328 automatically bounces off to drive the core rod 4322 and the mandrel 4241 to retreat towards the proximal end (for example, a direction indicated by an arrow 315 in Fig. 33), which indicates that threaded connection between the distal end of the mandrel 4241 and the shaft body 213 of the valve clip 20 has been released, and the valve clip 20 has been released.

When the disengagement control assembly 432 needs to be switched from the second state to the first state, the safety switch 4326 is pressed down, such that the safety switch 4326 is abutted against the core rod 4322 to form a whole, and the transmission member 4242 axially moves to drive the mandrel 4241 and the core rod 4322 to move synchronously.

Since the disengagement control assembly 432 is provided with the safety switch 4325, connection between the mandrel 4241 and the valve clip 20 may be released only after the safety switch 4325 is pulled up (that is, when the disengagement control assembly 432 is in the second state), which prevents misoperation and unexpected disengagement of the valve clip 20, and improves safety of surgery. The one-way gear 4324 only allows the core rod 4322 to drive the mandrel 4241 to rotate towards a direction of releasing threaded connection, such that the mandrel 4341 can be disengaged efficiently.

As shown in Figs. 1, 7 and 17, the valve clip delivery device 40 further includes an steerable sheath 60. The steerable sheath 60 includes an adjustable handle 62 and a sheath 64 connected with the adjustable handle 62 and controlled by the adjustable handle 62 to bend correspondingly. The catheter 44 is movably passed through the sheath 64, such that the distal end of the catheter 44 can be bent correspondingly in compliance with bending of the distal end of the sheath 64, so as to conform to the curved intervention path. The steerable sheath 60 further includes a connecting tube 66 connected with the adjustable handle 62. The connecting tube 66 is movably sleeved outside the catheter 44 and extends into the housing 422, and the connecting tube 66 is detachably connected with the housing 422. In some embodiments, the connecting tube 66 is detachably connected with the housing 422 by means of a mounting member 68. The mounting member 68 is preferably a claw screw, and the mounting member 68 can fix or unfix the connecting tube 66 to the housing 422. In an unfixed state, a distance between the valve clip 22 and the distal end of the steerable sheath 60 can be adjusted by pushing and pulling the connecting tube 66, such that the valve clip 20 reaches a predetermined position. After the adjustment above-mentioned, the connecting tube 66 can be fixed by using the mounting member 68, so as to maintain the position of the valve clip 20. Understandably, the mounting member 68 being the claw screw is not limited in the disclosure. The mounting member 68 can also be other types, as long as the connecting tube 66 can be detachably connected with the housing 422.

A working process of a valve clip system 100 is briefly described below. The valve clip 20 is delivered transcatheterly by the valve clip delivery device 40 to a vicinity of a mitral valve of a patient.

In an initial state, the first control rod 4232 is pushed forwards (towards a distal end) to keep the valve clip 20 in a self-locking state, and the two second control rods 4282 are pulled back towards a proximal end and limited, such that the two grasping members 28 are in a relatively close state. The safety switch 4326 is abutted against the core rod 4322, and the transmission member 4242 is integrated with the core rod 4322. An operator adjusts a bending degree of the sheath 64 by means of the adjustable handle 62, and the catheter 44 disposed in the sheath 64 conforms to the sheath 64 to deliver the valve clip 20 to a predetermined position near the mitral valve of the patient.

The first control rod 4232 is driven to move towards the proximal end, and the valve clip 20 is switched from the self-locking state to the unlocked state. That is, self-locking of the valve clip 20 is removed by means of the locking control assembly 423. The first guide portion 4230 is accommodated in the limiting groove 4238 and is abutted against a limiting step 4239 to keep the valve clip 20 in the unlocked state.

Then, the clamping members 25 of the valve clip 20 are controlled to be opened by means of the clamping control assembly 424. For example, the knob 4245 of the clamping control assembly 424 is rotated in the opposite direction (for example, a direction indicated by an arrow 303 in Fig. 23), such that the two clamping members 25 are relatively opened.

Then, each of the two grasping members 28 is controlled by means of the grasping control assembly 428 to capture the leaflet between the clamping member 25 and the grasping member 28. For example, referring to Fig. 30, one of the two second control rods 4282 can be pushed forwards towards the distal end to capture one leaflet. Another one of the two second control rods 4282 is pushed forwards towards the distal end to capture another leaflet.

Next, the two clamping members 25 are controlled by means of the clamping control assembly 424 to be closed, so as to clamp the anterior leaflet and the posterior leaflet of the mitral valve together. For example, the knob 4245 of the clamping control assembly 424 is rotated towards a positive direction (e.g., a direction indicated by an arrow 305 in Fig. 24), such that the two clamping members 25 are closed to clamp the leaflets.

Once the leaflets of the mitral valve are aligned edge to edge, the first fixing member 4236 is removed and the locking control wire 4231 is withdrawn from a body, the second fixing member 4286 is removed, and the grasping wire 4281 is withdrawn from the body.

The operator pulls up the safety switch 4326 (for example, a direction indicated by an arrow 313 in Fig. 33), such that the safety switch 4326 is disengaged from the core rod 4322. The release knob 4329 of the disengagement control assembly 432 is rotated (for example, a direction indicated by an arrow 314 in Fig. 33) to release connection between the mandrel 4241 and the shaft body 213 of the valve clip 20, the valve clip delivery device 40 is withdrawn from the patient's body, and the valve clip 20 is maintained at the mitral valve to achieve edge-to-edge repair.

Understandably, the valve clip system 100 of the disclosure may also implement edge-to-edge repair of a tricuspid valve in a transcatheter manner.

What are described above are merely preferred embodiments of the disclosure, and are not intended to limit the disclosure in any form. Although the disclosure has been disclosed in the above preferred embodiments, the disclosure is not limited to the preferred embodiments. Any person skilled in the art can make many possible changes and modifications to the technical solution of the disclosure by utilizing the method and technical content disclosed above or modify the technical solution of the disclosure to equivalent embodiments without departing from the scope of the technical solution of the disclosure. Hence, any simple alteration, equivalent change and modification which are made to the above-mentioned embodiments in accordance with the technical essence of the disclosure without departing from the content of the technical solution of the disclosure all fall within the scope of protection of the technical solution of the disclosure.

## Claims

1. A valve clip delivery device configured to deliver and control a valve clip, wherein the valve clip comprises at least two clamping members which are relatively opened and closed, and the valve clip is provided with a self-locking mechanism; wherein the valve clip delivery device comprises a handle and a catheter, wherein the handle comprises a housing, a clamping control assembly and a locking control assembly, wherein a proximal end of the catheter is fixedly connected with a distal end of the housing, the clamping control assembly and the locking control assembly are both disposed on the housing, the locking control assembly is connected with the self-locking mechanism and is configured to control the valve clip to be switched between an unlocked state and a self-locking state, and the clamping control assembly is configured to control the at least two clamping members to be opened or closed when the valve clip is in the unlocked state.

2. The valve clip delivery device as claimed in claim 1, wherein the locking control assembly comprises a locking control wire and a first control rod, wherein the first control rod is movably disposed in the housing, the locking control wire is connected with the first control rod and the self-locking mechanism, and a portion of the locking control wire located between the first control rod and the self-locking mechanism is movably disposed in the catheter; and the first control rod drives the locking control wire to move in a direction from the distal end of the housing to a proximal end of the housing, such that the valve clip is switched from the self-locking state to the unlocked state.

3. The valve clip delivery device as claimed in claim 2, wherein the locking control wire comprises a folded section and two connecting sections extending from two sides of the folded section respectively, wherein two first sub-cavities are provided in the catheter, a proximal end of one of the two connecting sections correspondingly movably passes through one of the two first sub-cavities and is connected with the first control rod, and the folded section is movably connected with the self-locking mechanism.

4. The valve clip delivery device as claimed in claim 2, wherein the locking control assembly further comprises a first sleeve, wherein the first sleeve is fixedly disposed in the housing, the first control rod is movably disposed in the first sleeve, a first guide portion is disposed on an outer wall of the first control rod, a second guide portion is disposed on an inner wall of the first sleeve, one of the first guide portion and the second guide portion is a guide groove extending in an axial direction of the first control rod, the other one of the first guide portion and the second guide portion is a guide protrusion, and the guide protrusion is slidably disposed in the guide groove.

5. The valve clip delivery device as claimed in claim 4, wherein the guide groove is disposed on the inner wall of the first sleeve, a limiting groove communicating with the guide groove is further disposed on the first sleeve, the limiting groove extends in a circumferential direction of the first sleeve and is provided with a limiting step, and the valve clip is kept in the unlocked state when the guide protrusion is disposed in the limiting groove and is abutted against the limiting step.

6. The valve clip delivery device as claimed in claim 2, wherein the locking control wire is made of at least one of ultra-high molecular weight polyethylene, nickel-titanium alloy and stainless steel.

7. The valve clip delivery device according to claim 2, wherein a distance of linear motion of the first control rod relative to the housing is 9mm to 13mm.

8. The valve clip delivery device as claimed in claim 1, wherein the valve clip delivery device further comprises at least two grasping control assemblies, and the valve clip further comprises at least two grasping members, wherein each of the at least two grasping control assemblies comprises a grasping wire and a second control rod, wherein the second control rod is movably disposed in the housing, the grasping wire is correspondingly connected with the second control rod and one of the at least two grasping members, and a portion of the grasping wire located between the second control rod and the one of the at least two grasping members is movably disposed in the catheter; and the at least two grasping control assemblies are configured to control the at least two grasping members to be relatively close to each other or opened radially.

9. The valve clip delivery device as claimed in claim 8, wherein the grasping wire comprises a folded section and two connecting sections extending from two sides of the folded section respectively, wherein at least four second sub-cavities are provided in the catheter, a proximal end of one of the two connecting sections correspondingly movably passes through one of the at least four second sub-cavities and is corresponding connected with the second control rod, and the folded section is movably connected with the grasping member.

10. The valve clip delivery device as claimed in claim 8, wherein the at least two grasping control assemblies are located on opposite sides of the housing respectively and on opposite sides of the catheter respectively.

11. The valve clip delivery device as claimed in claim 8, wherein a stroke of the second control rod is greater than or equal to 2πR×θ/360, wherein R is a length of each of the at least two grasping members, θ is an absolute value of a difference value between a first angle and a second angle, the first angle is an angle between each of the at least two grasping members and a central axis of the valve clip when the at least two grasping members are relatively close to each other, and the second angle is a maximum angle between one of the at least two grasping members and the central axis of the valve clip after the at least two grasping members opened radially.

12. The valve clip delivery device as claimed in claim 1, wherein the clamping control assembly comprises a mandrel and a transmission member, wherein a distal end of the mandrel is detachably connected with the valve clip, a proximal end of the mandrel movably passes through the catheter and is connected with the transmission member, and the transmission member is slidably connected with the housing in an axial direction of the housing; and in the unlocked state, the transmission member drives the mandrel to slide in the axial direction of the housing, such that the at least two clamping members of the valve clip are relatively opened or closed.

13. The valve clip delivery device as claimed in claim 12, wherein the clamping control assembly further comprises a constant-force gear and a knob configured to drive the constant-force gear to rotate, wherein the constant-force gear is in threaded connection with the transmission member, and the knob is sleeved on the constant-force gear;
when the knob rotates towards a positive direction and a driving force of the knob to the constant-force gear is less than a preset force value, the knob drives the constant-force gear to synchronously rotate towards the positive direction, such that the transmission member slides towards one end of the housing in the axial direction relative to the housing; and when the knob rotates towards the positive direction and the driving force of the knob to the constant-force gear is greater than or equal to the preset force value, the knob independently rotates relative to the constant-force gear; and
when the knob rotates towards an opposite direction of the positive direction, the knob drives the constant-force gear to synchronously rotate towards the opposite direction, such that the transmission member slides towards an other end of the housing in the axial direction relative to the housing.

14. The valve clip delivery device as claimed in claim 13, wherein the constant-force gear comprises a gear body and a plurality of toothed arms protruding from an outer wall of the gear body and disposed at intervals in a circumferential direction of the gear body, and an inner wall of the knob is provided with a plurality of stop portions matching the plurality of toothed arms in the circumferential direction; and the preset force value is a value of an acting force applied to the constant-force gear by the knob when each toothed arm relatively slides through each corresponding stop portion.

15. The valve clip delivery device as claimed in claim 14, wherein one end of the toothed arm is fixedly connected with the outer wall of the gear body, the other end of the toothed arm is a free end, at least part of the outer wall of the toothed arm extends in a circumferential direction of the gear body, and a gap is formed between the at least part of the outer wall of the toothed arm and the outer wall of the gear body.

16. The valve clip delivery device as claimed in claim 15, wherein the free end of the toothed arm comprises a first sliding guide surface and a first sliding stop surface disposed opposite the first sliding guide surface, and the stop portion of the knob comprises a second sliding guide surface and a second sliding stop surface disposed opposite the second sliding guide surface; when the knob rotates towards the positive direction, the first sliding guide surface is contacted with the second sliding guide surface; and when the knob rotates towards the opposite direction, the first sliding stop surface is contacted with the second sliding stop surface.

17. The valve clip delivery device as claimed in claim 12, further comprising a disengagement control assembly connected with the proximal end of the mandrel, wherein the disengagement control assembly is configured to disengage the mandrel from the valve clip.

18. The valve clip delivery device as claimed in claim 17, wherein the distal end of the mandrel is in threaded connection with the valve clip, and the disengagement control assembly comprises a core rod, a one-way gear and a safety switch, wherein the core rod is disposed in the transmission member and is fixedly connected with the proximal end of the mandrel, and the one-way gear is fixedly sleeved on the mandrel and is accommodated in the transmission member; and
the disengagement control assembly has a first state and a second state, wherein when the disengagement control assembly is in the first state, the safety switch is abutted against the core rod, and the core rod and the transmission member are connected into a whole; and when the disengagement control assembly is in the second state, the safety switch is separated from the core rod, and the core rod unidirectionally rotates relative to the transmission member, thereby driving the distal end of the mandrel to be disengaged from the valve clip.

19. The valve clip delivery device as claimed in claim 18, wherein the disengagement control assembly further comprises an elastic member, wherein the elastic member extends along an axial direction of the core rod and is held between the core rod and the transmission member.

20. The valve clip delivery device as claimed in claim 1, further comprising a steerable sheath, wherein the steerable sheath comprises an adjustable handle and a sheath connected with the adjustable handle and controlled by the adjustable handle to bend correspondingly, wherein the catheter is movably disposed in the sheath; and
the steerable sheath further comprises a connecting tube connected with the adjustable handle, wherein the connecting tube is movably sleeved outside the catheter and extends into the housing, and the connecting tube is detachably connected with the housing.
